# EUROPEAN PATENT APPLICATION

(11) **EP 3 267 347 A1**
(43) Date of publication of application: **10.01.2018**
(21) Application number: 16178379.0
(22) Date of filing: 07.07.2016
(51) Int. Cl.: G06F 19/28

(54) **ELECTRONIC PLATFORM FOR PROVIDING METHODS FOR THE INTERPRETATION OF NUCLEIC ACID SEQUENCES**

(71) Applicant: Siemens Healthcare GmbH, 91052 Erlangen (DE)
(72) Inventor: Cassarino, Antonino, 2340 Mödling (AT); Kremer, Andreas, 54292 Trier (DE); Krenn, Franz, 1090 Wien (AT); Posch, Andreas Emanuel, 1090 Wien (AT); Winkler, Erich, 2392 Grub (AT)

(57) **Abstract**

The present invention refers to methods and systems for executing an interpretation module (IM), which is dedicated to generate an automatic response for a respective medical question in relation to a sequencing dataset, comprising the following method steps:
- Accessing (41) an annotation database (ADB) with a specified query for the respective medical question;
- Extracting (43) and providing (44) a result annotation dataset (53) within the interpretation module (IM);
- Providing (45) a clinical output (58) via an output interface (57) in a formalized format;
- Storing (46) the clinical output (58) for the medical question in relation to the sequencing dataset and the applied computerized clinical guidelines in an encapsulated form (60) as a constant in form of program code,
wherein during all method steps it is ensured (47) that machine readable clinical guidelines, validated interpretation rules and technical quality criteria, which are stored in the interpretation module, are met.

## Description

The present invention lies within the field of bio-informatics and refers to a method and system for automated variant interpretation of a nucleic acid sequencing dataset for a specific medical question.

Machine-based, automatic interpretation of anomalies in genomic data requires robust and clinically validated applications and a quick and reliable transformation of new scientific data into clinical practice.

DNA sequencing is the process of determining the precise order of nucleotides within a DNA molecule. It includes any method or technology that is used to determine the order of the four bases - adenine, guanine, cytosine, and thymine - in a strand of DNA. The dataset may be provided in a so called FASTQ format (e.g. with a sequence of the before mentioned bases, like: "CTGATGTG..."). The advent of rapid DNA sequencing methods leads to bulky data, which need to be processed reliably and the processing results need to be reproducible. Next-generation sequencing (NGS) refers to high-throughput nucleic acid (e.g. DNA and RNA) sequencing technologies. Millions or billions of DNA strands can be sequenced in parallel, yielding substantially more throughput and minimizing the need for cost intensive further methods.

Knowledge of DNA sequences has become indispensable for basic biological research, and in numerous applied fields such as medicine, biotechnology, forensic biology, virology and biological systematics.

Generally, clinical diagnostic information processing based on next generation sequencing can be divided in 3 steps:
(i) pre-analytics and sequencing,
(ii) raw data processing and variant calling, and,
(iii) variant interpretation.

Step (i) starts with sampling and ends with the generation of sequencing raw data.

Variant calling in step (ii), deals with raw data processing as well as alignment, assembly, identification of personal sequence variants compared to the human reference genome and generation of variant call format (VCF) variant file. Variant calling is an important procedure for targeted, whole-exome and whole-genome sequencing, and in some cases also for RNA-sequencing.

Next-generation sequencing generally produces reads or read pairs. To compare the DNA of the sequenced sample to its reference sequence, it is necessary to find the corresponding part of that sequence for each read in the sequencing data. This is called aligning or mapping the reads against the reference sequence. Once this is done, it is possible to look for variations (e.g. SNPs) within the sample.

Generally, alignments can be used for different purposes, e.g. for whole-genome sequencing (by sequence all DNA from an organism and map it to the appropriate reference sequence, to find genetic variation), for targeted sequencing (by capturing just the exomic DNA before sequencing) and for transcriptome sequencing (RNA-Sequencing).

By virtue of increased complexity of the raw sequencing data, a shift in genetic testing has been accompanied by new challenges in sequence interpretation.

Variant interpretation in step (iii) subsequently identifies clinically relevant variants by functional annotation using biomedical knowledge bases (e.g. ClinVar or Genometrax2, a commercial collection including several variation and mutation databases such as HGMD or COSMIC).

Biomedical knowledge bases, however, are continuously updated as the knowledge about the clinical relevance of variants is constantly evolving. Additionally, the human reference genome is regularly updated by The Genome Reference Consortium to better represent human diversity and provide more accurate references for genome analysis.

Genotyping of BRCA1 (breast cancer 1, early onset), a human tumor suppressor gene can be employed to determine the likelihood of getting breast cancer. The table below shows the changes in genomic coordinates based on the Genome Build. Novel releases in (e.g. annotation) databases, however, affect genomic coordinates, as exemplarily shown for BRCA1 in the table below:

| Gene | Genome Build | Chromosome | Start | End |
|---|---|---|---|---|
| BRCA1 | GRCh37.p13 | 17 | 41196312 | 41277500 |
| BRCA1 | GRCh38 | 17 | 43044295 | 43125483 |

Consequently, automated interpretation of sequencing results not only demands complying with predefined standard operation procedures throughout step (i) to (iii) but may also lead to spurious results upon upgrading of biomedical knowledge bases.

Existing solutions for variant interpretation either use local installations of biomedical knowledge bases or perform interpretation of uploaded VCF files via web applications. In both cases, it is the responsibility of the user to ensure compatibility of annotation sources with pre-analytics, sequencing and variant calling procedures. Therefore, current practice commonly requires manual curation of results, which is not suitable for automated decision support in a clinical setting, especially since quality criteria including robustness, accuracy, reproducibility and independency of interpretation rules cannot be guaranteed. Additionally, uploading genomic data for interpretation by web tools may represent a data safety breach while maintenance and timely updating of local copies of biomedical knowledge bases is a challenging and time consuming task.

Interpretation of anomalies in genomic data requires robust, clinically validated applications and a quick transformation of new scientific knowledge into everyday clinical practice.

In this context, the problem is the absence of technical processes and infrastructures which can convey medical rules for interpretation of genomic data from a research institution to routine operations in a quick and secure way.

Based on the challenges, mentioned above, it is an object of the present invention to provide automatic decision support for different medical questions. This should include a result in form of a clinical output for nucleic acid sequence interpretation. The clinical output should be reproducible and independent of interpretation rules and the state of annotation databases. Further, the underlying technical system should assist the software developer for generating interpretation applications which automatically meet the standard operating procedures and quality guidelines and the system has to assure that uniform quality requirements are met.

This object is solved by the subject matter according to the accompanying independent claims. Preferred embodiments are subject of the dependent claims, the description and the figures.

According to a first aspect the present invention refers to a method for development, validation and distribution of a set of interpretation modules, wherein an interpretation module is provided as computer application and is constructed to automatically generate a response to a medical question in relation to a sequencing dataset, comprising the following method steps:
- Determining a standard operating procedure for the generation of the nucleic acid sequencing raw data starting from a patient sample and/or for the nucleic acid sequencing workflow in relation to the medical question and analytical technologies to be used for analysis of the sequencing dataset;
- Determining technical quality criteria/parameter in relation to the medical question and analytical technologies to be used for analysis of the sequencing dataset;
- Developing the interpretation module according to the determined standard operating procedure and the technical quality criteria/parameter
- Validating the developed interpretation module
- Storing the validated interpretation module for distribution to a target system.

The medical question and/or the sequencing dataset may serve as input for the interpretation module in an operational phase of the interpretation module (software application).

The method may be computer-implemented.

In the following, a definition of terms is given.

The sequencing dataset is a nucleic sequence data set. It may be processed by pre-analytics and sequencing and may be provided in a pre-defined format (e.g. FASTQ format). The sequencing dataset may be based on raw data processing and variant calling. The result of variant calling may be provided as files in a variant call format (VCF), which may serve as input for the interpretation module(s). However, variant calling may also be subject to an interpretation module, so that only pre-processed data, e.g. in form of raw sequence data may serve as input for the interpretation module. Further, it is also possible to input a medical question in the interpretation module, referring to a question how to provide a variant calling file for further analysis.

The medical question is a question of clinical relevance for a specific patient. The medical question may be related to a specific disease, to a finding, to a diagnosis or to validate an existing diagnosis. The medical question is typically related to a sequencing dataset. For example, a medical question could be "Are there any genetic evidences for the disease abc?", "Is there a mutation at position xyz?", "What is the genotype for the drug-metabolizing enzymes xyz?", "What targeted therapy has the highest success chance based on the RNA expression profile?".

The medical question may be determined by providing input data on a user interface. The user may select pre-defined buttons in order to be assisted in defining the medical question. Further, it is possible to automatically import a medical question from a computer based information system, like e.g. HIS/KIS, PACS, etc.

The annotation database is an electronic database or a memory with an interface to such a database, which is permanently updated with the latest scientific reports and knowledge. It is possible to combine several different annotation databases as one virtual common or overall database cluster.

The interface between the interpretation module and the annotation database may be provided according to a proprietary or standard protocol. Preferably, the queries for accessing the annotation database are formatted according to a uniform or consistent protocol over all different interpretation modules. After accessing the annotation database with the query a result annotation dataset will be generated and provided and forwarded to the respective interpretation module. In response to receiving the result annotation dataset at the interpretation module, the interpretation module may - optionally - be configured to further process the result annotation dataset in order to generate a clinical output. However, it is also possible that the result annotation dataset will not be further processed and, thus, serves also as a clinical output.

The clinical output is an electronic dataset, which is characterized in that it only consists of the requested data which are relevant for the specific medical question. The clinical output will be provided in a formalized format. The clinical output is generated by means of a formalized interpretation (e.g. rule-based, model-based, etc.) of the result annotation dataset.

The standard operating procedure is an electronic dataset, which represents the technical processes for nucleic acid sequencing analysis. It may also comprise technical quality criteria. The standard operating procedures or SOP may, for example, refer to sampling and measurements procedures, including all experimental protocols from sampling to sequencing. It comprises data, indicating, which samples were taken, how the nucleic acid samples were isolated, purified and processed prior to sequencing, a summary of the instrumentation used, library preparation strategy, the region of interest being studied and/or enrichment and amplification methodologies, etc. The technical processes typically comprise a sequence of processing steps, which need to be executed, like accessing reference and annotation databases, executing alignment and variant calling algorithms, data processing and analysis, interpretation protocols.

The technical quality criteria/parameter are provided as an electronic dataset, which represents, the quality criteria which need to be applied for all or a subset of technical processes (sampling, sequencing, analyzing, etc.). Technical quality criteria may include the amount and purity of the nucleic acid, the nucleic acid library size, average and minimal sequencing coverage, average and minimal base quality, ... ).

The clinical guideline(s) is an electronic dataset (which typically consists of a plurality of subsets and different entries), which represents the clinical implications for different identified genetic variants. The Clinical Guidelines are published by medical societies. One example of such a clinical guideline e.g. refers to molecular testing guidelines for colorectal cancer. More detailed information for this guideline may be found under the link: http://www.amp.org/committees/clinical practice/documents/2015 0327CRCMMDraftRecommendationsforOCP-UPDATEDfinaldraft 001.pdf.

The clinical guidelines do also comprise general recommendations and/or standards for interpretation of sequence variants. As an example it is referred to the American college for medical genetics http://www.nature.com/gim/journal/v17/n5/full/gim201530a.html.

According to a further aspect, the present invention refers to a method for operating a computer based platform for providing - and executing as a need-based service, if required - interpretation modules, which have been developed, validated and stored according to a method of the directly preceding method claim.

According to a further aspect, the present invention refers to a (preferably computer implemented) method for executing an interpretation module, which is dedicated to a medical question and in particular for generating an automatic response to the respective medical question in relation to a sequencing dataset, comprising the following method steps:
- Accessing an annotation database with a specified query for the respective medical question;
- Extracting and providing a result annotation dataset within the interpretation module;
- Providing a clinical output via an output interface in a formalized format;
- Storing the clinical output for the medical question in relation to the sequencing dataset and the applied computerized clinical guidelines in an encapsulated form as a constant in form of program code,
wherein during all method steps it is ensured that machine readable clinical guidelines, which are stored in the interpretation module, are met.

In a preferred embodiment, the extracted result annotation dataset is formalized according to a standard operation procedure (in the following, also abbreviated as "SOP").

In a preferred embodiment, the stored clinical output is uniquely assigned to its medical question for the respective sequencing dataset (the medical question refers to) and its result annotation dataset and the clinical guidelines which have been applied.

In general, the medical question and the clinical output (answer) are linked in an encapsulated element for later access and later execution (for generation of encapsulated elements/modules). The answer may consist of the result annotation dataset, the clinical output, the guidelines, which have been applied, the standard operating procedures and the state of the respective databases.

In a preferred embodiment, the sequencing dataset comprises:
- A genomic data sequence, which needs to be analyzed; and/or an indication of a genomic data sequence, e.g. as a link to a respective entry of the genomic data sequence in a database
- Variant files (e.g. VCF files) for the genomic data sequence.

In a preferred embodiment, the result annotation dataset only consists of interpretation data, being relevant for the medical question. This is due to the fact, that only the relevant data are capsuled during generation of the interpretation module, e.g. region of interest, reference genome, observed allele frequencies for genomic variations in the region of interest, etc.

In a preferred embodiment, the stored clinical output comprises an execution date or time. This has the advantage, that the generation to the clinical output as result of the interpretation module and all the states / conditions of the SOP, Guidelines and databases may be traceable and tracked over time. Thus, the overall state (comprising the state of the databases, the state of the Guidelines, and the state of the standard operating procedures and the state of the technical quality criteria) may be "frozen".

In a preferred embodiment, the stored clinical output automatically expires after a predefined expiration time interval. The expiration date guarantees a regular verification of the actuality of the interpretation rules. Upon expiry, database updates may be triggered and an updated Interpretation Module constructed following the development - validation workflow. Modified or revalidated Interpretation Modules can be provided for an IM-Target System again via the distribution-infrastructure in a quick and secure way. The locally retrievable expiration date within the interpretation module enables to warn about usage of possibly out-dated Interpretation Modules without a need for availability of additional management systems.

In a preferred embodiment, the stored clinical output is signed, in particular digitally, by a creator. This has the technical advantage, that the quality of the system and method may be improved, as responsibilities may be tracked. It is also possible that the clinical output comprises a checksum for further improving quality by assuring correct transfer of data.

According to a further aspect the present invention refers to an interpretation module, which is dedicated to a respective medical question and in particular for generating an automatic response to the medical question in relation to a sequencing dataset, comprising:
- An input interface for receiving the sequencing dataset or a medical question in relation to the sequencing dataset;
- An annotation interface for accessing a database with a specified query for the medical question and for receiving a result annotation dataset;
- A Memory for storing technical guideline data, which are provided in a machine readable format;
- A processor for executing an interpretation application for automatically providing clinical output as a response to the medical question on the basis of the result annotation dataset;
- A user interface and
- An output interface for representation of the clinical output.

According to a further embodiment of the present invention, the interpretation module comprises a worldwide unique identification feature. Thus, each interpretation module may be uniquely accessed by its identification feature. The identification feature may be an identification number.

According to a further embodiment of the present invention, the interpretation module comprises an expiration date.

According to a further aspect, the present invention refers to a system for generation, distribution and execution of interpretation modules. The interpretation modules have been mentioned above in different possible embodiments. The system comprises:
- A central server with:
   - A development system, which is adapted to develop interpretation modules and which comprises a runtime environment;
   - A validation system, which is adapted to validate developed interpretation modules;
   - A storage and distribution system, which is adapted to store and distribute validated interpretation modules;
- A target system, which is adapted to invoke and execute the interpretation module.

Generally, all modules of the system may be - at least temporarily - in data exchange with each other, in particular the central server and the target system are linked via network connection or respective interfaces.

The development system and the validation system may be combined to a development and validation system. In this development and validation system, necessary tools and/or relevant reference data and/or validation data are provided. Alternatively, the performance parameter may be requested by the validation system (e.g. based on regulatory/clinical guidelines or best practice recommendations).

The target system may invoke the interpretation module upon request on a need-based request. The target system may execute the interpretation module either centrally on the central server (via web-services) or locally on the local processor of the target system. The target system may be hosted locally on a local site, e.g. in a computer based node of a client, whereas the server may be hosted on a central server. The storage and distribution system may be hosted centrally as well or may be combined with the development and validation system as well.

According to another aspect, the invention further refers to a computer program, having program instructions embodied therewith, the program instructions executable by a processor to cause technical operations. The technical operations comprise the steps mentioned before with respect to the method for development, validation and distribution, the method for operating a computer based platform and/or the method for executing an interpretation module, with the respective method steps.

According to another aspect, the invention further refers to a computer program product. The computer program product may be embodied in a computer readable storage medium having program instructions embodied therewith. The program instructions, being executable by a processor to cause operations, as mentioned before with respect to the computer program. The computer program product may be embodied on a central server and/or on a target system and several internal or external databases. The computer program product may in addition to the computer program comprise development material, a runtime system and/or databases, libraries etc. The computer program product may be distributed among several computer instances.

### BRIEF DESCRIPTION OF THE DRAWINGS

Fig. 1 shows a block diagram of a development and distribution system for interpretation modules according to an embodiment of the invention.
Fig. 2 shows a block diagram of a development and validation system with a runtime environment for interpretation modules according to an embodiment of the invention.
Fig. 3 depicts a flow chart for a method for development, validation and distribution of interpretation modules according to an embodiment of the invention.
Fig. 4 depicts a flow chart for a method for executing an interpretation module according to an embodiment of the invention.
Fig. 5 represents elements and modules of an interpretation module according to an embodiment of the invention and
Fig. 6 shows a data structure for the clinical output according to an embodiment of the invention.

### DETAILED DESCRIPTION OF EMBODIMENTS

The present invention is based on the encapsulation of medical questions into Interpretation Modules IM and describes the technical features of a runtime environment IM-R for the development, validation, distribution and usage of such Interpretation Modules IM for clinical decision support.

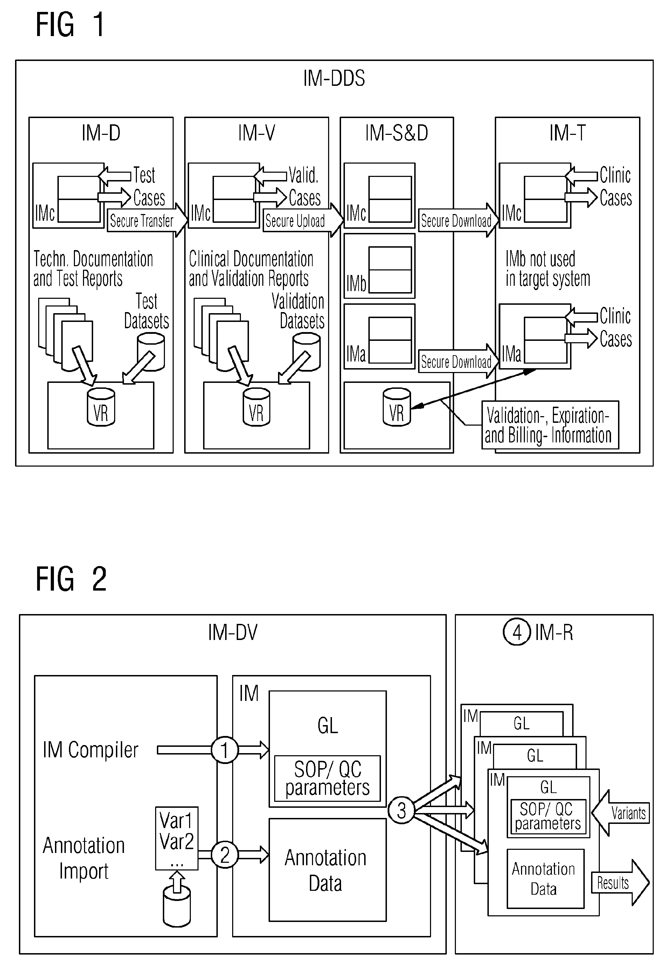

**Fig. 1** shows an interpretation module development and distribution system IM-DDS, which serves as a platform for creation, distribution and usage of Interpretation Modules IM in a schematic representation. According to an embodiment the interpretation module development and distribution system IM-DDS comprises:
- An interpretation module development system IM-D,
- An interpretation module validation system IM-V,
- An interpretation module store & distribution system IM-S&D and
- An interpretation module target system IM-T.

The (set of) interpretation modules IM are hosted by the systems of the interpretation module platform.

An interpretation module IM represents a medical question which is answered with help of genetic data.

An interpretation module IM corresponds to the encapsulated, formal representation of a medical question, which requires genomic tests. It includes the following characteristics:
i. The interpretation module IM contains annotation data relevant for nucleic acid sequence interpretation in the context of the medical question. This information is extracted from validated biomedical knowledge bases ADB and included as a constant in form of program code within the interpretation module IM.
ii. The interpretation module IM contains computerized clinical guidelines GL in from of program code.
iii. The interpretation module IM contains interfaces to genomic data files (e.g. VCF files) as well as to patient and specimen data relevant to the medical question (e.g. age, sex, ethnicity, etc.).
iv. The interpretation module IM contains interfaces to represent the clinical output (e.g. PDF, HTML, HTML5, etc.)
v. The interpretation module IM contains a standard operation procedure SOP for
   (i) pre-analytics and sequencing,
   (ii) raw data processing and variant calling, and,
   (iii) variant interpretation designed to the medical question
vi. The interpretation module may only be executed after the user confirms compliance with the standard operation procedure SOP.

The IM-Development System IM-D represents an environment for university hospitals or research facilities, where Interpretation Modules are developed.

The IM-Validation System IM-V represents an environment for university hospitals or research facilities, where interpretation modules are validated. This system also contains a database for all the data which is relevant for the reproducibility of a validation.

The IM-Store and Distribution System IM-S&D represents a central platform for storing, administration and distribution of validated Interpretation Modules IM. This system also contains a database for all the data which is relevant for the reproducibility of a validation and queries can be called via web services.

In the IM-Target System IM-T the Interpretation Modules IM are used for interpretation of genome data. The target system IM-T can be part of a Hospital Information System (HIS) or represent a stand-alone NGS-application which is optionally integrated in a HIS via Interfaces.

As can be seen in Fig. 1, the interpretation module (e.g. IMc) is created and technically tested in the IM-Development System IM-D. The test datasets are saved in a Validation Repository VR. The distribution-infrastructure allows a secure transfer of the interpretation module IM and the corresponding test datasets to the IM-Validation System IM-V.

In the IM-Validation System IM-V the results of the interpretation module (e.g. IMc) get clinically validated. The relevant validation datasets are also saved in the Validation Repository VR. The distribution-infrastructure allows a secure upload of the interpretation module IM and the corresponding validation datasets to the IM-Store and Distribution System IM-S&D.

The IM-Store and Distribution System IM-S&D provides validated Interpretation Modules IM (e.g. IMa, IMb, IMc) and corresponding validation information for usage.

Validated Interpretation Modules IM can be loaded securely into the IM-Target System IM-T and used for the analysis of genome data. E.g. in the depicted IM-Target System IM-T only the interpretation modules IMa and IMc are used.

**Fig. 2** shows an interpretation module development and validation system IM-DV according to another embodiment of the invention. However, it has to be mentioned, that all different embodiments of the present invention may be combined.

Fig. 2 shows encapsulated Interpretation Modules IM, integrating Functional Sequence Annotation and computerized clinical guidelines GL for Clinical Decision Support. Reference numeral 1 in Fig. 2 represents a development of an interpretation module stub. Reference numeral 2 in Fig. 2 represents an extraction and formalization of annotation data for subsequent import in the interpretation module IM as constant data type. Reference numeral 3 in Fig. 2 represents a finalization of the programming and distribution of the interpretation module IM. Reference numeral 4 in Fig. 2 represents a testing and clinical validation of Interpretation Modules IM in a runtime environment IM-R.

By combining computerized clinical guidelines GL with constant, annotation data along with formalized description of standard operation procedures SOP in Interpretation Modules IM, Interpretation Modules IM are independent of updates in biomedical knowledge bases /the human reference genome. Interpretation Modules IM do not need to be re-validated upon updates in biomedical knowledge bases. Interpretation Modules IM may be developed, tested, validated, distributed and applied independently.

Interpretation Modules IM may only be executed for the intended use and in compliance with the validated standard operation procedures SOP.

Consequently, only the introduction of Interpretation Modules IM facilitates testing and clinical validation of interpretation rules incorporating sequence annotation data and thus facilitates robustness and accuracy of clinical decision support systems for sequencing results.

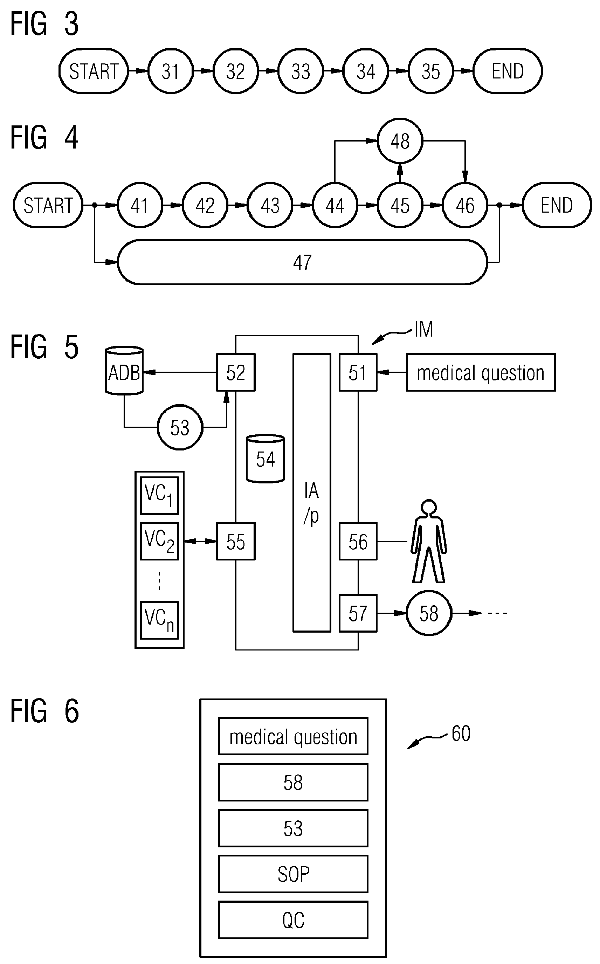

**Fig. 3** is a flow chart according to an embodiment for executing a method for development, validation and distribution of a specific interpretation module of a set of interpretation modules IM. After starting, in step 31 standard operating procedures SOP are determined for defining analytical technologies to be used for analysis of the sequencing dataset. In step 32 technical quality criteria / parameter QC are determined, which are also to be used for the analysis. In step 33 the interpretation module IM is generated. In step 34 the developed interpretation module IM is validated before it will be stored in step 35 in order to be distributed to a target system IM-T. Fig. 3 generally refers to a development phase, in which the interpretation modules are to be developed.

The development phase is adapted to automatically assist the interpretation module software developer in complying with predefined rules, quality criteria and parameter, SOPs and Guidelines GL.

**Fig. 4** is a flow chart according to an embodiment for executing a specific interpretation module IM, which has been developed in the development phase, which has been explained above with respect to Fig. 3. Thus, Fig. 4 refers to a later, subsequent so called operational phase for execution of the developed interpretation modules IM. After starting, in step 41 a medical question is determined. This could be executed by receiving input via a user interface 56 or by importing the medical question from another computer based instance. In step 42 a formalized query if formed in order to access an annotation database ADB for the received medical question. In step 43 data is extracted in the annotation database ADB and a result annotation dataset 53 is imported to the interpretation module IM and is provided there in step 44. The result annotation dataset 53 may be further processed in order to provide a clinical output 58 in step 45 via an output interface 57. The clinical output is provided in a formalized format, which may be pre-defined in the preparation phase. This has the technical effect and serves to make it possible to compare different results from different interpretation modules IM. The clinical output 58 is stored in step 46 in an encapsulated form 60 as a constant in form of program code. After providing the extracted result annotation dataset 53 in step 44 or after providing the clinical output 58 in step 45, the data may be formalized according to the standard operating procedures SOP in step 48. This has the technical effect that the result data may be provided in a uniform data structure, which makes it easier to automatically further process the data via different computer based applications. During all steps, which have been mentioned with respect to Fig. 4, it is assured that the machine readable guidelines GL are met, which is represented in Fig. 4 in step 47. After storing the clinical output in step 46 the method may be re-executed or may end.

**Fig. 5** shows a block diagram for an interpretation module IM according to a preferred embodiment of the present invention. The interpretation module IM has an input interface 51 for receiving the medical question in relation to a specific diagnostic question, in particular with respect to a genomic data sequence. The interpretation module IM further consists of an annotation interface 52 for accessing an annotation database for extracting relevant annotation data as a result annotation dataset 53. The interpretation module IM further consists of a guideline memory 54 for storing clinical guidelines GL, which have to be applied for analysis of the sequencing data in relation to answering the medical question. The interpretation module IM comprises a variant caller interface 55 for accessing different variant callers VC, e.g. VC1, VC2, VC3. The interpretation module IM further comprises a user interface 56 and an output interface 57 for providing the clinical output 58. The interpretation module IM comprises as its kernel a processor P for executing the interpreting application IA for providing the clinical output 58.

**Fig. 6** shows a special data structure for providing the clinical output 58 in form of a data capsule element 60. The clinical output 58 is preferably provided and stored in an encapsulated form. The encapsulated form 60 assigns the respective medical question to its clinical output 58 - as the main result - but in addition stores the sequencing dataset, the result annotation dataset 53, the guidelines GL with the standard operation procedures SOP as well as the quality criteria parameter, which have been applied for analysis of the sequencing dataset. Thus, the status of the databases ADB, VC and the status of the guidelines GL, with the SOPs and the quality parameter QC, which have been applied are combined and stored in a special format. The respective datasets are assigned, so that the actual status can be "frozen" and may be accessible for future queries.

According to an embodiment of the invention a description of the patient with meta data (age, sex, ethnic data, anamnesis data) and the medical question are received or imported in a first step. After that, sampling and measurement procedures including all experimental protocols from sampling to sequencing are executed. According to the invention, it is tracked, which samples were taken, how the nucleic acid samples were isolated, purified and processed prior to sequencing, a summary of the instrumentation used, library preparation strategy, the region of interest being studied, enrichment and amplification methodologies, etc. Further, the raw sequence read data including quality scores are stored as well. Moreover, the processing pipeline, including used reference and annotation databases are tracked. Further, alignment and variant calling algorithms, data processing and analysis, interpretation protocols are stored together with the clinical output 58 in the encapsulated format 60.

According to the invention, the overall system for interpretation of genetic data is divided into single Interpretation Modules (IMs) which can separately be developed, validated and used in clinical routine with help of the described distribution infrastructure.

An interpretation module IM has the following technical features for the described process:
1) it contains rules, data and Interfaces which are used for the analysis and interpretation of genome data, which is relevant for the medical question, and for providing result data 53, 58.
2) it contains a worldwide unique identification number, a title, a creator and a version.
3) it contains descriptions of the respective implemented medical question, descriptions of the implemented rules as well as references to the corresponding known scientific literature.
4) it contains data about the runtime environment IM-R of the IM-Development system IM-D.
5) It contains an expiration date.
6) It contains a checksum and a digital signature of the creator.

The implementation of medical questions in single Interpretation Modules IM allows a fast reaction to the scientific progress and a quick introduction of new scientific knowledge into routine operations.

The usage of interfaces for genome data, patient data and presentation of results as well as the encapsulation of annotation data with interpretation rules leads to Interpretation Modules IM which are separately exchangeable and transportable and which are independent from annotation databases ADB.

The distribution-infrastructure and the digital signature allow a quick and secure transfer of Interpretation Modules from the IM-Validation System via the IM-Store and Distribution System to the IM-Target System. Due to the ensured IM-identity a revalidation in the IM-Target system is avoided.

Data about the runtime environment of the IM-Validation System stored within the Validation Repository allow an automated test in the IM-Target System IM-T if the interpretation module IM is validated and executable for the respective technical runtime environment of the IM-Target System IM-T.

The locally retrievable data within the interpretation module IM about the runtime environment of the IM-Development System IM-D enables to block the usage of interpretation modules in incompatible runtime environments without a need for availability of additional management systems.

Via the IM-Store and Distribution System IM-S&D and due to the worldwide unique identification it is possible to invalidate Interpretation Modules IM even before expiration date and block their application in the IM-Target System IM-T, if necessary.

Usage data may be exchanged between IM-Target IM-T system and IM-Store. Commercial issues like e.g. billing and support can be carried out via the IM-Store and Distribution System IM-S&D.

In the IM-Target System IM-T it is possible to locally access the descriptions in the interpretation module about the respective implemented medical question, the implemented rules as well as the corresponding known scientific literature without a need for availability of additional documentation systems.

From the IM-Target System IM-T it is possible to access additional information from the IM-Store and Distribution System IM-S&D, regarding further scientific publications for the medical question which were published after the clinical validation.

Generally, a variety of in silico tools, both publicly and commercially available, can aid in the interpretation of sequence variants. However, the algorithms used by each tool may differ but can include determination of the effect of the sequence variant at the nucleotide and amino acid level, including determination of the effect of the variant on the primary and alternative gene transcripts, other genomic elements, as well as the potential impact of the variant on the protein. This makes it difficult, to compare the respective results and to provide reliable results over time for different statuses of the databases or the processing rules. The invention solves this problem, by providing a formalized approach and a platform for uniformly processing and storing the data in an encapsulated format 60.

According to the invention it is possible to provide special application-depending and varying quality metrics for the assay, including - but not limited to:
a) Minimum and average quality of base
b) Minimum and average criteria for depth of coverage and uniformity of coverage
c) Minimum and average percent of variant reads to make a heterozygous call

According to the invention it is possible to provide specific procedures for verifying the assay with new lots/shipments of reagents.

According to the invention it is possible to provide a specific policy, defining that will and what will not be reported from targeted sequencing, /WGS results. If incidental findings are reported, indicate the criteria for reporting of incidental findings.

According to the invention it is possible to provide specific standard operating procedures SOP for all processes within the NGS assay including template preparation, library preparation, target enrichment, sequencing, data analysis and interpretation of results.

According to the invention it is possible to provide a specific flowchart to show the workflow through these procedures, SOP, and quality control/quality assurance procedures.

Generally, there are multiple variant databases (eg. ClinVar, DMuDB, Exome Variants Server, 1000 genomes, dbSNP, HGMD, DECIPHER etc.) which vary according to the population (including age, disease status, ethnicity), variant type, data quality and annotation (including pathogenicity status). An understanding of the structure and content of these databases is essential in order to utilize them effectively. Therefore, the present invention defines a computer based platform with a formalized structure, how to interpret and use the content of the databases for genetic analysis.

It is essential to store the clinical output file 58 from the variant annotation step (eg. vcf file) and depending on regulatory aspects and laboratory policy one may choose to also retain the FastQ, SAM or BAM files in order to re-analyse the read data in the future. These data are stored together with a log of the informatics processing that was applied to the raw data in order to make the sequence and/or mapping files.

The presented method describes extracting only the genomic information relevant to the specific medical question from collections of variations and mutations in biomedical knowledge bases and subsequent linking with computerized clinical guidelines GL as well as technical operation procedures SOP in embedded interpretation modules to
i. guarantee robustness, accuracy and reproducibility of clinical decision support systems by introducing genomic data as constants or in encapsulated form;
ii. facilitate distribution of interpretation modules IM via central services.

The foregoing description of various embodiments of the invention has been presented for the purposes of illustration and description. It is not intended to be exhaustive or to limit the invention to the precise form disclosed. Many modifications and variations are possible in light of the above teaching. It is intended that the scope of the invention be limited not by this detailed description, but rather by the claims appended hereto. The above specification, examples and data provide a complete description of the manufacture and use of the composition of the invention. Since many embodiments of the invention can be made without departing from the spirit and scope of the invention, the invention resides in the claims herein after appended.

## Claims

1. A method for development, validation and distribution of a set of interpretation modules (IM), wherein an interpretation module (IM) is provided as computer application and is constructed to automatically generate a response to a medical question in relation to a sequencing dataset which serves as input for the interpretation module (IM) in an operational phase of the interpretation module, comprising the following method steps:
- Determining (31) a standard operating procedure (SOP) for the sequencing workflow in relation to the medical question and analytical technologies to be used for analysis of the sequencing dataset;
- Determining (32) technical quality criteria parameters (QC) in relation to the medical question and analytical technologies to be used for analysis of the sequencing dataset;
- Developing (33) the interpretation module (IM) according to the determined standard operating procedure (SOP) and the technical quality criteria parameter (QC)
- Validating (34) the developed interpretation module (IM)
- Storing (35) the validated interpretation module (IM) for distribution to a target system.

2. A method for operating a computer based platform for providing interpretation modules (IM), which have been developed (33), validated (34) and stored (35) according to a method of the directly preceding method claim.

3. A method for executing an interpretation module (IM), which is dedicated to a medical question and in particular for generating an automatic response to the respective medical question in relation to a sequencing dataset, comprising the following method steps:
- Accessing (41) an annotation database (ADB) with a specified query for the respective medical question;
- Extracting (43) and providing (44) a result annotation dataset (53) within the interpretation module (IM);
- Providing (45) a clinical output (58) via an output interface (57) in a formalized format;
- Storing (46) the clinical output (58) for the medical question in relation to the sequencing dataset and the applied computerized clinical guidelines in an encapsulated form (60) as a constant in form of program code,
wherein during all method steps it is ensured (47) that machine readable clinical guidelines, which are stored in the interpretation module, are met.

4. Method according to claim 3, wherein the extracted result annotation dataset (53) and/or the clinical output (58) is formalized (48) according to a standard operation procedure (SOP).

5. Method according to any of the preceding method claims 3 to 4, wherein the stored clinical output (58) is uniquely assigned to its medical question for the sequencing dataset and its result annotation dataset (53) and the clinical guidelines which have been applied.

6. Method according to any of the preceding method claims 3 to 5, wherein the sequencing dataset comprises:
- A DNA sequence,
- A RNA sequence and/or
- Variant files for the genomic data sequence.

7. Method according to any of the preceding method claims 3 to 6, wherein the result annotation dataset (53) only consists of interpretation data relevant for the medical question.

8. Method according to any of the preceding method claims 3 to 7, wherein the stored clinical output (58) comprises an execution time.

9. Method according to any of the preceding method claims 3 to 8, wherein the stored clinical output (58) automatically expires after a predefined expiration time interval.

10. Method according to any of the preceding method claims 3 to 9, wherein the stored clinical output (58) is signed by a creator.

11. An interpretation module (IM), which is dedicated to a respective medical question and in particular for generating an automatic response to a medical question in relation to a sequencing dataset, comprising:
- An input interface (51) for receiving the sequencing dataset or a question in relation to the sequencing dataset
- An annotation interface (52) for accessing a database (ADB) with a specified query for the medical question and for receiving a result annotation dataset (53)
- A Memory for storing technical guideline (SOP, QC);
- A processor (P) for executing an interpretation application (IA) for automatically providing a response to the medical question
- A user interface (56) and
- An output interface (57) for representation of the clinical output (58).

12. An interpretation module (IM) according to the directly preceding claim, wherein the interpretation module (IM) comprises a worldwide unique identification feature.

13. An interpretation module (IM) according to any of the preceding claims directed to the interpretation module (IM), wherein the interpretation module (IM) comprises an expiration date.

14. System for generation, distribution and execution of interpretation modules (IM) according to any of the preceding claims referring to the interpretation module (IM), comprising:
- A central server with a development and validation system (IM-DV) with:
- A development system (IM-D), which is adapted to develop interpretation modules (IM) and which comprises a runtime environment (IM-R);
- A validation system (IM-V), which is adapted to validate developed interpretation modules (IM);
- A storage and distribution system (IM-S&D), which is adapted to store and distribute validated interpretation modules (IM);
- A target system (IM-T), which is adapted to invoke and execute the interpretation module (IM).
